# EUROPÄISCHE PATENTSCHRIFT

(11) **EP 3 082 739 B1**
(45) Veröffentlichungstag und Bekanntmachung des Hinweises auf die Patenterteilung: **07.11.2018**
(21) Anmeldenummer: 14799661.5
(22) Anmeldetag: 23.09.2014
(51) Int. Cl.: A61K 8/36, A61Q 5/10, A61K 8/73, A61K 8/22, A61K 8/04

(54) **SCHAUMSTABILISIERTE HAARFÄRBEMITTEL**
HAIR DYE COMPOSITION IN THE FORM OF A STABILISED FOAM
COLORANTS CAPILLAIRES À MOUSSE STABILISÉE

(30) Priorität: 17.12.2013 DE 102013226283
(43) Veröffentlichungstag der Anmeldung: 26.10.2016
(73) Patentinhaber: Henkel AG & Co. KGaA, 40589 Düsseldorf (DE)
(72) Erfinder: SCHÖPGENS, Jürgen, 41366 Schwalmtal (DE); MÜLLER, Burkhard, 40221 Düsseldorf (DE)
(86) Internationale Anmeldenummer: PCT/DE2014/200500
(87) Internationale Veröffentlichungsnummer: WO 2015/090290

(56) Entgegenhaltungen:
- EP-A1- 2 277 499
- EP-A1- 2 277 600
- EP-A2- 1 342 465
- WO-A1-2011/075652
- JP-A- H10 167 938
- US-A1- 2009 089 940

## Beschreibung

Die Erfindung betrifft Mittel zum Färben, gemäß den vorliegenden Ansprüchen, die geeignet sind, durch spezielle Applikationsvorrichtungen in Form eines stabilen Schaums aufgetragen zu werden, ein Färbeverfahren unter Zuhilfenahme der Mittel und der Applikationsvorrichtung sowie ein entsprechendes Kit zur Färbung von keratinhaltigen Fasern.

Für das Färben von keratinhaltigen Fasern kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet. Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt. Als Kupplerkomponenten werden in der Regel m-Phenylendiaminderivate, Naphthole, Resorcin und Resorcinderivate, Pyrazolone, m-Aminophenole und substituierte Pyridinderivate verwendet.

Zumeist besitzen oxidative Färbemittel zur Stabilisierung der Farbstoffvorprodukte während der Lagerung und zur Reaktionsbeschleunigung während der oxidativen Anwendung einen alkalischen pH-Wert, der mit Alkalisierungsmitteln, wie Alkanolaminen, Ammoniak oder anorganischen Basen, eingestellt wird. Insbesondere Ammoniak ermöglicht dabei zwar gute Färbeergebnisse, offenbart jedoch aufgrund seines Geruchs und Reizpotentials für Haut und Schleimhäute auch Nachteile für den Anwender.

Oxidative Färbemittel bestehen üblicherweise aus zwei Komponenten, deren Mischung ausreichend zähflüssig ist, damit sie bequem auf die Haare aufgetragen werden kann und dabei nicht tropft oder verläuft. Darüber hinaus gab es häufig Versuche, andere Konfektionierungsformen zu entwickeln. So wurde vorgeschlagen, dünnflüssigere Färbemittel mit speziellen Applikatorsystemen auf die Haare aufzubringen oder Färbemittel als Schaum zu applizieren. Bei der Schaumapplikation ist insbesondere der Einsatz von Aerosolschäumen verbreitet.

Allerdings besteht in jüngerer Zeit auch das Bedürfnis, auf den Einsatz von Treibgasen verzichten zu können. Ein weiteres Problem bei der Schaumapplikation ist die Stabilisierung der Schäume. Die Beschaffenheit von Schäumen ist dann als ideal zu bezeichnen, wenn bei der Produktabgabe ein fester, stabiler Schaum entsteht, der ein geschmeidiges Gefühl hinterlässt und auf dem Haar nur langsam bricht. Häufig ist aber zu beobachten, daß die ausgebrachten Schäume wenig stabil sind und schnell wieder in sich zusammenfallen, so daß eine dünnflüssige, tropfende Lösung zurückbleibt. Andererseits ist es auch wesentlich, daß der Schaum trotzdem die Haare gut benetzt, so daß ein guter Farbauftrag stattfinden kann. Die Schaumstabilität wird insbesondere durch die Anwesenheit größerer Mengen an Salzen und Farbstoff(vorprodukt)en negativ beeinflusst. Aus der JP10-167938 A sind bereits schaumförmige Haarbehandlungsmittel bekannt, die als besonders geeignete Tenside für eine stabile Schaumbildung von sauren, wasserstoffperoxid-haltigen Haarbehandlungsmitteln betainische Tenside vom Typ der Alkyldimethylbetaine und der (Fettsäureamidoalkyl)dialkylbetaine wie Cocoamidopropylbetain offenbaren. Cocoamidopropylbetain ist auch als geeignetes Tensid mit hohem Tensidgehalt in Tönungsschäumen auf Basis direktziehender Farbstoffe in WO 2006/066642(A1) beschrieben WO 2011/075652 offenbart ebenfalls schaumförmige Körperpflegemittel.

Es zeigte sich jedoch in umfangreichen Untersuchungen, daß gerade Schaumhaarfarben besondere Herausforderungen an die Formulierungskünste der Produktentwickler stellen. So muß der Schaum einerseits stabil genug sein, um eine problemlose Anwendung zu gewährleisten, andererseits muß er während der Anwendung zügig brechen, um eine gleichmäßige und intensive Benetzung der Haare sicherzustellen.

Zerfällt der Schaum allerdings zu schnell, so läuft die flüssige Anwendungsmischung aus den Haaren und von der Kopfhaut in den Stirn-, Nacken- und Schläfernbereich und führt dort zu störenden Verfärbungen an der Haut bzw. der Kleidung oder gar zu Irritationen der Augen.

Aufgabe der vorliegenden Erfindung war es daher, oxidative Färbemittel für die Schaumapplikation, ohne Verwendung von Treibgasen zu optimieren, so daß die oben genannten Nachteile überwunden werden können. Insbesondere sollten stabile Färbeschäume ohne Leckage-Probleme bereitgestellt werden.

Es wurde überraschenderweise gefunden, daß sich die vorstehend genannten Probleme durch spezielle oxidative Färbemittel lösen lassen. Die Stabilität bleibt auch bei höheren Salzkonzentrationen erhalten. Außerdem ermöglichen die erfindungsgemäßen Mittel intensivere Farbergebnisse und deutlich haltbare Farbergebnisse als bisher bekannte Schaumzubereitungen. Schließlich sind die Mittel geruchsarm.

Ein erster Gegenstand der vorliegenden Erfindung sind daher Mittel zum Färben keratinischer Fasern, enthaltend mindestens zwei getrennt voneinander verpackte Zubereitungen (A) und (B) sowie gegebenenfalls eine weitere getrennt von (A) und (B) verpackte Zubereitung (C), die unmittelbar vor der Anwendung zu einer Anwendungsmischung vermischt werden, wobei
i. Zubereitung (A)
   a) mindestens ein Oxidationsfarbstoffvorprodukt und
   b) mindestens ein Kaliumsalz einer Fettsäure enthält und
ii. Zubereitung (B) fließfähig ist und - bezogen auf ihr Gewicht -
   a) mindestens ein Oxidationsmittel und
   b) Xanthan
   enthält.

Ein zweiter Gegenstand der vorliegenden Erfindung sind Verfahren zum Färben keratinischer Fasern, bei dem mindestens zwei getrennt voneinander verpackte Zubereitungen (A) und (B) sowie gegebenenfalls eine weitere getrennt von (A) und (B) verpackte Zubereitung (C), unmittelbar vor der Anwendung zu einer Anwendungsmischung vermischt und aus einem Spender in Form eines Schaumes ausgebracht werden, dadurch gekennzeichnet, daß
i. Zubereitung (A)
   a) mindestens ein Oxidationsfarbstoffvorprodukt und
   b) mindestens ein Kaliumsalz einer Fettsäure enthält und
ii. Zubereitung (B) fließfähig ist und - bezogen auf ihr Gewicht -
   c) mindestens ein Oxidationsmittel und
   d) Xanthan
   enthält.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie beispielsweise Regeneratcellulose, Nitro-, Alkyl- oder Hydroxyalkyl- oder Acetylcellulose verwendet werden.

Die erfindungsgemäßen Mittel enthalten die Wirkstoffe in einem kosmetisch akzeptablen Träger. Dieser kosmetische Träger ist bevorzugt wässrig, alkoholisch oder wässrig-alkoholisch. Ein wässriger Träger enthält im Sinne der Erfindung mindestens 40 Gew.-%, insbesondere mindestens 50 Gew.-% Wasser. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten, sofern sie die Schaumbildung und -stabilität nicht übermäßig negativ beeinflussen. Bevorzugt sind dabei alle wasserlöslichen organischen Lösungsmittel. Bevorzugte erfindungsgemäße Mittel sind dadurch gekennzeichnet, daß sie zusätzlich ein nichtwässriges Lösungsmittel enthalten, wobei bevorzugte erfindungsgemäße Mittel das Lösungsmittel in einer Konzentration von 0,1 bis 30 Gew.-%, bevorzugt in einer Konzentration von 1 bis 20 Gew.-%, ganz besonders bevorzugt in einer Konzentration von 2 bis 10 Gew.-%, jeweils bezogen auf das Mittel, enthalten.

Als erste erfindungswesentliche Komponente enthalten die erfindungsgemäßen Mittel in der Zubereitung (A) mindestens ein Oxidationsfarbstoffvorprodukt. Vorzugsweise enthält das Mittel eine oder mehrere Entwicklerkomponenten sowie gegebenenfalls eine oder mehrere Kupplerkomponenten.

Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 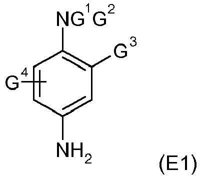 wobei
- G¹ steht für ein Wasserstoffatom, einen C₁-C₄-Alkylrest, einen C₁-C₄)-Monohydroxyalkylrest, einen C₂-C₄-Polyhydroxyalkylrest, einen C₁-C₄-Alkoxy-(C₁-C₄)-alkylrest, einen 4-Aminophenylrest oder einen C₁-C₄-Alkylrest, der mit einer stickstoffhaltigen Gruppe, einem Phenyl- oder einem 4-Aminophenylrest substituiert ist;
- G² steht für ein Wasserstoffatom, einen C₁-C₄-Alkylrest, einen C₁-C₄-Monohydroxyalkylrest, einen C₂-C₄-Polyhydroxyalkylrest, einen C₁-C₄-Alkoxy-(C₁-C₄)-alkylrest oder einen C₁-C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁-C₄-Alkylrest, einen C₁-C₄-Monohydroxyalkylrest, einen C₂-C₄-Polyhydroxyalkylrest, einen C₁-C₄-Hydroxyalkoxyrest, einen C₁-C₄-Alkoxy-(C₁-C₄)-alkylrest, einen C₁-C₄-Acetylamino-alkoxyrest, einen Mesylamino-(C₁-C₄)-alkoxyrest oder einen C₁-C₄-Carbamoylaminoalkoxyrest;
- G⁴ steht für ein Wasserstoffatom, ein Halogenatom, einen C₁-C₄-Alkylrest oder einen C₁-C₄-Alkoxy-(C₁-C₄)-alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Besonders bevorzugte p-Phenylendiamine der Formel (E1) werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(2-hydroxyethyl)-p-phenylendiamin, 4-N,N-Bis-(2-hydroxyethyl)-amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N,N-(Ethyl,2-hydroxyethyl)-p-phenylendiamin, N-(2,3-Dihydroxypropyl)-p-phenylendiamin, N-(4-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Erfindungsgemäß ganz besonders bevorzugte p-Phenylendiaminderivate der Formel (E1) sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie deren physiologisch verträglichen Salze.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze, 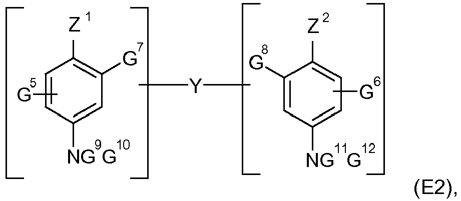 wobei
- Z¹ und Z² stehen unabhängig voneinander für einen Hydroxyl- oder NH₂-Rest, der gegebenenfalls durch einen C₁-C₄-Alkylrest, durch einen C₁-C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁-C₈-Alkoxyreste substituiert sein kann, oder eine direkte Bindung,
- G⁵ und G⁶ stehen unabhängig voneinander für ein Wasserstoff- oder Halogenatom, einen C₁-C₄-Alkylrest, einen C₁-C₄-Monohydroxyalkylrest, einen C₂-C₄-Polyhydroxyalkylrest, einen C₁-C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁-C₄-Alkylrest, mit der Maßgabe, daß die Verbindungen der Formel (E2) nur eine Verbrückung Y pro Molekül enthalten.

Bevorzugte zweikernige Entwicklerkomponenten der Formel (E2) werden insbesondere aus mindestens einer der folgenden Verbindungen ausgewählt: N,N'-Bis-(2-hydroxyethyl)-N,N'-bis-(4-aminophenyl)-1,3-diamino-propan-2-ol, N,N'-Bis-(2-hydroxyethyl)-N,N'-bis-(4-aminophenyl)ethylendiamin, N,N'-Bis-(4-aminophenyl)tetramethylendiamin, N,N'-Bis-(2-hydroxyethyl)-N,N'-bis-(4-aminophenyl)tetramethylendiamin, N,N'-Bis-(4-(methylamino)phenyl)tetramethylendiamin, N,N'-Diethyl-N,N'-bis-(4-amino-3-methylphenyl)ethylendiamin, Bis-(2-hydroxy-5-aminophenyl)methan, N,N'-Bis-(4-aminophenyl)-1,4-diazacycloheptan, N,N'-Bis-(2-hydroxy-5-aminobenzyl)piperazin, N-(4-Aminophenyl)-p-phenylendiamin und 1,10-Bis-(2',5'-diaminophenyl)-1,4,7,10-tetraoxadecan sowie ihre physiologisch verträglichen Salze. Ganz besonders bevorzugte zweikernige Entwicklerkomponenten der Formel (E2) werden ausgewählt unter N,N'-Bis-(2-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines deren physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3), 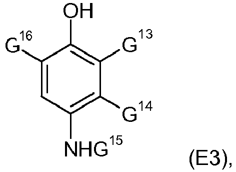 wobei
- G¹³ steht für ein Wasserstoffatom, ein Halogenatom, einen C₁-C₄-Alkylrest, einen C₁-C₄-Mono-hydroxyalkylrest, einen C₂-C₄-Polyhydroxyalkylrest, einen C₁-C₄-Alkoxy-(C₁-C₄)-alkylrest, einen C₁-C₄-Aminoalkylrest, einen Hydroxy-(C₁-C₄)-alkylaminorest, einen C₁-C₄-Hydroxyalkoxyrest, einen C₁-C₄-Hydroxyalkyl-(C₁-C₄)-aminoalkylrest oder einen Di-[(C₁-C₄)-alkyl]amino-(C₁-C₄)-alkylrest, und
- G¹⁴ steht für ein Wasserstoff- oder Halogenatom, einen C₁-C₄-Alkylrest, einen C₁-C₄-Monohydroxyalkylrest, einen C₂-C₄-Polyhydroxyalkylrest, einen C₁-C₄-Alkoxy-(C₁-C₄)-alkylrest, einen C₁-C₄-Aminoalkylrest oder einen C₁-C₄-Cyanoalkylrest,
- G¹⁵ steht für Wasserstoff, einen C₁-C₄-Alkylrest, einen C₁-C₄-Monohydroxyalkylrest, einen C₂-C₄-Polyhydroxyalkylrest, einen Phenylrest oder einen Benzylrest, und
- G¹⁶ steht für Wasserstoff oder ein Halogenatom.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethylaminomethyl)phenol, 4-Amino-2-(1,2-dihydroxyethyl)phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze. Besonders bevorzugte Verbindungen der Formel (E3) sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen.

Besonders bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Besonders bevorzugte Pyrazol-Derivate sind insbesondere die Verbindungen, die ausgewählt werden unter 4,5-Diamino-1-methylpyrazol, 4,5-Diamino-1-(β-hydroxyethyl)-pyrazol, 3,4-Diaminopyrazol, 4,5-Diamino-1-(4-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol sowie deren physiologisch verträglichen Salze.

Bevorzugte Pyrazolopyrimidine sind Pyrazolo[1,5-a]pyrimidine. Unter den Pyrazolo[1,5-a]pyrimidinen sind wiederum Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethyl-pyrazolo[1,5-a]pyrimidin-3,5-diamin, 3-Aminopyrazolo[1,5-a]pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]pyrimidin-3-ylamino)ethanol, 2-[(3-Aminopyrazolo[1,5-a]pyrimidin-7-yl)-(2-hydroxy-ethyl)-amino]-ethanol, 2-[(7-Aminopyrazolo[1,5-a]-pyrimidin-3-yl)-(2-hydroxy-ethyl)-amino]-ethanol, 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-a]pyrimidin sowie ihre physiologisch verträglichen Salze und ihre tautomeren Formen, wenn ein tautomers Gleichgewicht vorhanden ist, besonders bevorzugt.

Ganz besonders bevorzugte Entwicklerkomponenten werden ausgewählt, aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin sowie deren physiologisch verträglichen Salzen.

Die Entwicklerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, daß bei Verwendung mindestens einer Entwicklerkomponente zusätzlich mindestens eine Kupplerkomponente zum Einsatz kommt.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Diese Gruppen stehen durch ein Doppelbindungssystem in Konjugation.

Erfindungsgemäße Kupplerkomponenten werden bevorzugt ausgewählt den Klassen von m-Aminophenol und/oder dessen Derivaten, m-Diaminobenzol und/oder dessen Derivaten, o-Diaminobenzol und/oder dessen Derivaten, Naphthalinderivaten mit mindestens einer Hydroxygruppe, Dibeziehungsweise Trihydroxybenzol und/oder deren Derivaten, Pyridinderivaten, Pyrimidinderivaten, Monohydroxyindol-Derivaten und/oder Monoaminoindol-Derivaten, Monohydroxyindolin-Derivaten und/oder Monoaminoindolin-Derivaten, Pyrazolonderivaten, wie 1-Phenyl-3-methylpyrazol-5-on, Morpholinderivate, wie 6-Hydroxybenzomorpholin oder 6-Amino-benzomorpholin, Chinoxalinderivaten, wie 6-Methyl-1,2,3,4-tetrahydrochinoxalin, sowie Gemischen aus zwei oder mehrer Verbindungen aus einer oder mehrerer dieser Klassen.

Bevorzugte m-Aminophenol-Kupplerkomponenten werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Di-ethylamino)-phenol, N-Cyclopentyl-3-aminophenol, 1,3-Dihydroxy-5-(methylamino)-benzol, 3-Ethyl-amino-4-methylphenol, 2,4-Dichlor-3-aminophenol und deren physiologisch verträglichen Salzen.

Bevorzugte m-Diaminobenzol-Kupplerkomponenten werden ausgewählt aus mindestens einer Verbindung aus der Gruppe, die gebildet wird aus 2-(2,4-Diaminophenoxy)ethanol, 1,3-Bis-(2,4-diaminophenoxy)propan, 1-Methoxy-2-amino-4-(2'-hydroxyethylamino)benzol, 1,3-Bis-(2,4-diamino-phenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}-amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol und deren physiologisch verträglichen Salzen.

Bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und deren physiologisch verträglichen Salzen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 2,6-Dihydroxy-3,4-dimethylpyridin, 3,4-Diaminopyridin, 2-(2-Methoxyethyl)amino-3-amino-6-methoxypyridin, 2-(4'-Methoxyphenyl)amino-3-aminopyridin und deren physiologisch verträglichen Salzen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Bevorzugte Indolderivate werden ausgewählt aus mindestens einer Verbindung der Gruppe, die gebildet wird aus 4-Hydroxyindol, 6-Hydroxyindol und 7-Hydroxyindol und deren physiologisch verträglichen Salzen.

Bevorzugte Indolinderivate werden ausgewählt aus mindestens einer Verbindung der Gruppe, die gebildet wird aus 4-Hydroxyindolin, 6-Hydroxyindolin und 7-Hydroxyindolin und deren physiologisch verträglichen Salzen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxy-pyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen.

Erfindungsgemäß besonders bevorzugte Kupplerkomponenten werden ausgewählt unter m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2-Hydroxyethyl)amino-2-methylphenol, 3-(Diethylamino)phenol, N-Cyclopentyl-3-aminophenol, 1,3-Dihydroxy-5-(methylamino)benzol, 3-Ethylamino-4-methylphenol, 2,4-Dichlor-3-aminophenol, 2-(2,4-Di-aminophenoxy)ethanol, 1,3-Bis-(2,4-diaminophenoxy)propan, 1-Methoxy-2-amino-4-(2-hydroxy-ethylamino)benzol, 1,3-Bis-(2,4-diaminophenyl)propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, Resorcinmonomethylether, 2-Methylresorcin, 5-Methylresorcin, 2,5-Dimethylresorcin, 2-Chlorresorcin, 4-Chlorresorcin, Pyrogallol, 1,2,4-Trihydroxybenzol, 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 2,6-Dihydroxy-3,4-dimethylpyridin, 3,4-Diaminopyridin, 2-(2-Methoxyethyl)amino-3-amino-6-methoxypyridin, 2-(4'-Methoxyphenyl)amino-3-aminopyridin, 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin, 2,3-Dihydroxynaphthalin, 4-Hydroxyindol, 6-Hydroxyindol, 7-Hydroxyindol, 4-Hydroxyindolin, 6-Hydroxyindolin, 7-Hydroxyindolin, 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin oder Gemischen dieser Verbindungen oder deren physiologisch verträglichen Salzen.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so daß Entwicklerkomponenten und Kupplerkomponenten in einem Mol-Verhältnis von 1:0,5 bis 1:3, insbesondere 1:1 bis 1:2, stehen können.

Zur weiteren Nuancierung der resultierenden Farbtöne kann es erfindungsgemäß bevorzugt sein, dem Mittel weiterhin mindestens einen direktziehenden Farbstoff zuzusetzen. Hierbei handelt es sich um Farbstoffmoleküle, die direkt auf das Substrat aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Heutzutage sind direktziehende Farbstoffe üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%. Direktziehende Farbstoffe können in anionische, kationische und nichtionische direktziehende Farbstoffe unterteilt werden.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 sowie Tetrabromphenolblau und Bromphenolblau bekannten Verbindungen.

Bevorzugte kationische direktziehende Farbstoffe sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden. Die Verbindungen, die auch unter den Bezeichnungen Basic Yellow 87, Basic Orange 31 und Basic Red 51 bekannt sind, sind ganz besonders bevorzugte kationische direktziehende Farbstoffe.

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, daß die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu und Alkannawurzel enthalten sind.

Weiterhin hat es sich als vorteilhaft erwiesen, wenn die erfindungsgemäßen Mittel zur weiteren Nuancierung zusätzlich eine oder mehrere Farbstoffvorstufen naturanaloger Farbstoffe enthalten. Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Besonders gut als Vorstufen naturanaloger Haarfarbstoffe geeignet sind Derivate des 5,6-Dihydroxyindolins. Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure. Als Vorstufen naturanaloger Haarfarbstoffe hervorragend geeignet sind weiterhin Derivate des 5,6-Dihydroxyindols. Bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure.

Als zweite erfindungswesentliche Komponente enthalten die erfindungsgemäßen Mittel in der Zubereitung (A) mindestens ein Kaliumsalz einer Fettsäure.

Besonders geeignet sind erfindungsgemäß die Kaliumsalze folgender Fettsäuren: Capronsäure (Hexansäure), Önanthsäure (Heptansäure), Caprylsäure (Octansäure), Pelargonsäure (Nonansäure), Caprinsäure (Decansäure), Laurinsäure (Dodecansäure), Myristinsäure (Tetradecansäure), Palmitinsäure (Hexadecansäure), Margarinsäure (Heptadecansäure), Stearinsäure (Octadecansäure), Arachinsäure (Eicosan-/Icosansäure), Behensäure (Docosansäure), Lignocerinsäure (Tetracosansäure), Cerotinsäure (Hexacosansäure), Montansäure (Octaacosansäure), Melissinsäure (Triacontansäure), Undecylensäure ((10Z)- Undeca- 10- ensäure), Myristoleinsäure ((9Z)- Tetradeca-9- ensäure), Palmitoleinsäure ((9*Z*)- Hexadeca- 9- ensäure), Petroselinsäure ((6*Z*)- Octadeca- 6-ensäure), Ölsäure ((9*Z*)- Octadeca- 9- ensäure), Elaidinsäure ((9*E*)- Octadeca- 9- ensäure), Vaccensäure ((11*E*)- Octadeca- 11- ensäure), Gadoleinsäure ((9*Z*)- Eicosa- 9- ensäure), Icosensäure ((11*Z*)- Eicosa- 11- ensäure), Cetoleinsäure ((11*Z*)- Docosa- 11- ensäure), Erucasäure ((13*Z*)-Docosa- 13- ensäure), Nervonsäure ((15*Z*)- Tetracosa- 15- ensäure), Linolsäure ((9*Z*,12*Z*)- Octadeca-9,12- diensäure), Alpha-Linolensäure ((9*Z*,12*Z*,15*Z*)- Octadeca- 9,12,15- triensäure), Gamma-Linolensäure ((6*Z*,9*Z*,12*Z*)- Octadeca- 6,9,12- triensäure), Calendulasäure ((8*E*,10*E*,12*Z*)- Octadeca-8,10,12- triensäure), Punicinsäure ((9*Z*,11*E*,13*Z*)- Octadeca- 9,11,13- triensäure), Alpha-Elaeostearinsäure ((9*Z*,11*E*,13*E*)- Octadeca- 9,11,13- triensäure), Beta-Elaeostearinsäure ((9*E*,11*E*,13*E*)- Octadeca- 9,11,13- triensäure), Arachidonsäure ((5*Z*,8*Z*,11*Z*,14*Z*)- Eicosa- 5,8,11,14-tetraensäure), Timnodonsäure ((5*Z*,8*Z*,11*Z*,14*Z*,17*Z*)- Eicosa- 5,8,11,14,17- pentaensäure), Clupanodonsäure ((7*Z*,10*Z*,13*Z*,16*Z*,19*Z*)- Docosa- 7,10,13,16,19- pentaensäure), Cervonsäure ((4*Z*,7*Z*,10*Z*,13*Z*,16*Z*,19*Z*)- Docosa- 4,7,10,13,16,19- hexaensäure).

Besonders bevorzugte erfindungsgemäße Mittel sind dadurch gekennzeichnet, daß die Zubereitung (A) eines oder mehrere der folgenden Kaliumsalze enthält: Kaliumcapronat, Kaliumönanthat, Kaliumcaprylat, Kaliumpelargonat, Kaliumcaprinat, Kaliumlaurinat, Kaliummyristinat, Kaliumpalmitinat, Kaliummargarinat, Kaliumstearinat, Kaliumarachinat, Kaliumbehenat, Kaliumlignocerinat, Kaliumcerotinat, Kaliummontanat, Kaliummelissinat, Kaliumundecylenat, Kaliummyristoleinat, Kaliumpalmitoleinat, Kaliumpetroselinat, Kaliumoleat, Kaliumelaidinat, Kaliumvaccenat, Kaliumgadoleinat, Kaliumicosenat, Kaliumcetoleinat, Kaliumerucaat, Kaliumnervonat, Kaliumlinolat, Kalium-alpha-Linolenat, Kalium-gamma-Linolenat, Kaliumcalendulat, Kaliumpunicinat, Kalium-alpha-Elaeostearinat, Kalium-beta-Elaeostearinat, Kaliumarachidonat, Kaliumtimnodonat, Kaliumclupanodonat, Kaliumcervonat.

Ganz besonders bevorzugte erfindungsgemäße Mittel enthalten in Zubereitung (A) mindestens ein Kaliumsalz mindestens einer ungesättigten Fettsäure. Hier sind bevorzugte erfindungsgemäße Mittel sind dadurch gekennzeichnet, daß die Zubereitung (A) Kaliumpalmitoleinat und/oder Kaliumpetroselinat und/oder Kaliumoleat und/oder Kaliumgadoleinat und/oder Kaliumicosenat und/oder Kaliumcetoleinat und/oder Kaliumerucaat und/oder Kaliumnervonat und/oder Kaliumlinolat und/oder Kalium-alpha-Linolenat und/oder Kalium-gamma-Linolenat und/oder Kaliumcalendulat enthält.

Unabhängig davon, ob eines oder mehrere Kaliumsalze eingesetzt werden, sind erfindungsgemäß besonders bevorzugte Mittel und Verfahren dadurch gekennzeichnet, daß die Zubereitung (A) - bezogen auf ihr Gewicht - 0,1 bis 10 Gew.-%, vorzugsweise 0,15 bis 7,5 Gew.-%, weiter bevorzugt 0,2 bis 6 Gew.-%, besonders bevorzugt 0,25 bis 5,5 Gew.-% und insbesondere 0,3 bis 5 Gew.-% mindestens eines Kaliumsalz mindestens einer Fettsäure enthält.

Äußerst bevorzugte erfindungsgemäße Mittel enthalten nur Kaliumsalze von Fettsäuren und sind frei von Natriumsalzen von Fettsäuren.

Die Zubereitungen (A) sind vorzugsweise alkalisch, wobei besonders bevorzugte erfindungsgemäße Mittel und Verfahren dadurch gekennzeichnet sind, daß die Zubereitung (A) einen pH-Wert von 8 bis 12, vorzugsweise von 8,5 bis 11,5 und insbesondere von 9 bis 11 aufweist.

Vorzugsweise wird dabei als Alkalisierungsmittel mindestens ein Alkanolamin eingesetzt.

Erfindungsgemäß einsetzbare Alkanolamine werden bevorzugt ausgewählt aus Alkanolaminen aus primären, sekundären oder tertiären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethyl-ethanolamin, Methylglucamin, Triethanolamin, Diethanolamin und Triisopropanolamin. Erfindungsgemäß ganz besonders bevorzugte Alkanolamine werden ausgewählt aus der Gruppe 2-Aminoethan-1-ol (Monoethanolamin), 2-Amino-2-methylpropan-1-ol, 2-Amino-2-methyl-propan-1,3-diol und Triethanolamin. Besonders bevorzugte Mittel enthalten als Alkanolamin mindestens Monoethanolamin.

Bevorzugt sind die Alkanolamine in einer Menge von 0,05 bis 20 Gew.-%, insbesondere von 0,5 bis 15 Gew.-%, jeweils bezogen auf das Gesamtgewicht des anwendungsbereiten Mittels, enthalten.

Besonders bevorzugte erfindungsgemäße Mittel und Verfahren sind dadurch gekennzeichnet, daß die Zubereitung (A) - bezogen auf ihr Gewicht - 0,1 bis 15 Gew.-%, vorzugsweise 0,5 bis 10 Gew.-%, weiter bevorzugt 2 bis 9 Gew.-%, besonders bevorzugt 3 bis 8 Gew.-% und insbesondere 4 bis 7 Gew.-% mindestens eines Alkanolamins aus primären, sekundären oder tertiären Aminen mit einem C₂-C₆-Alkylgrundkörper, der mindestens eine Hydroxylgruppe trägt, bevorzugt ausgewählt aus der Gruppe, die gebildet wird aus 2-Aminoethan-1-ol (Monoethanolamin), 3-Aminopropan-1-ol, 4-Amino-butan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethyl-ethanolamin, Triethanolamin, Diethanolamin und Triisopropanolamin und besonders bevorzugt ausgewählt aus Monoethanolamin, enthält.

Zubereitung (A) kann darüber hinaus weitere Inhaltsstoffe enthalten. Diese werden weiter unten beschrieben.

Die erfindungsgemäßen Mittel und Verfahren sind weiter dadurch gekennzeichnet, daß die Zubereitung (B) mindestens ein Oxidationsmittel enthält.

In einer besonderen Ausführungsform enthalten die Zubereitungen (B) gemäß der Erfindung Wasserstoffperoxid als Oxidationsmittel.

Die Konzentration einer Wasserstoffperoxid-Lösung in der Oxidationsmittelzubereitung (B) wird einerseits von den gesetzlichen Vorgaben und andererseits von dem gewünschten Effekt bestimmt. Vorzugsweise enthalten die Zubereitungen (B) bezogen auf ihr Gewicht Wasserstoffperoxid (berechnet als 100%iges H₂O₂) in Mengen von 0,1 bis 30 Gew.-%, bevorzugt von 0,5 bis 20 Gew.-%, besonders bevorzugt von 1 bis 15 Gew.-% und insbesondere bevorzugt von 3 bis 8 Gew.-%.

Besonders bevorzugte erfindungsgemäße Mittel oder Verfahren sind dadurch gekennzeichnet, daß die Zubereitung (B) - bezogen auf ihr Gewicht - 0,1 bis 15 Gew.-%, vorzugsweise 0,25 bis 12 Gew.-%, weiter bevorzugt 0,5 bis 10 Gew.-% und insbesondere 3 bis 8 Gew.-% Wasserstoffperoxid (berechnet als 100%iges H₂O₂) enthält.

Erfindungsgemäß bevorzugte anwendungsbereite Mittel sind dadurch gekennzeichnet, daß sie, bezogen auf das Gesamtgewicht des anwendungsbereiten Mittels, 0,01 bis 12 Gew.-%, vorzugsweise 0,1 bis 10 Gew.-%, besonders bevorzugt 1 bis 8 Gew.-% Wasserstoffperoxid enthalten.

Zur Stabilisierung des Wasserstoffperoxid kann der pH-Wert der Zubereitung (B) bevorzugt auf pH 3 bis 5, besonders bevorzugt auf pH 3,5 bis 4,5 und insbesondere bevorzugt auf pH 3,8 bis 4,2 eingestellt werden.

Besonders bevorzugte erfindungsgemäße Mittel oder Verfahren sind dadurch gekennzeichnet, daß die Zubereitung (B) einen pH-Wert von 3 bis 5, besonders bevorzugt von pH 3,5 bis 4,5 und insbesondere bevorzugt von pH 3,8 bis 4,2 aufweist.

Als zweite wesentliche Komponente enthält die Zubereitung (B) Xanthan.

Insbesondere bevorzugt ist die Verwendung des Xanthan-Biopolymers, welches unter dem Handelsnamen Keltrol CG-SFT der Firma Kelco vertrieben wird. Weitere geeignete handelsübliche Xanthane sind beispielsweise Kelzan (Xanthan-Biopolymer, Kelco), Xanthan FN (Xanthan-Biopolymer, Jungbunzlauer), Keltrol z.B. Keltrol CG-T (Xanthan-Biopolymer, Kelco) oder das vorstehend genannte Keltrol CG-SFT (Xanthan-Biopolymer, Kelco) angeboten.

Erfindungsgemäß besonders bevorzugte Mittel oder Verfahren sind dadurch gekennzeichnet, daß die Zubereitung (B) - bezogen auf ihr Gewicht - 0,01 bis 4 Gew.-%, vorzugsweise 0,02 bis 3,5 Gew.-%, weiter bevorzugt 0,03 bis 3 Gew.-%, besonders bevorzugt 0,04 bis 2,5 Gew.-% und insbesondere 0,05 bis 2 Gew.-% Xanthan enthält.

Erfindungsgemäß besonders bevorzugte Mittel oder Verfahren sind dadurch gekennzeichnet, daß die Zubereitung (B) - bezogen auf ihr Gewicht - 0,01 bis 4 Gew.-%, vorzugsweise 0,02 bis 3,5 Gew.-%, weiter bevorzugt 0,03 bis 3 Gew.-%, besonders bevorzugt 0,04 bis 2,5 Gew.-% und insbesondere 0,05 bis 2 Gew.-% Xanthan enthält.

Sowohl Zubereitung (A) (siehe oben) als auch Zubereitung (B) können weitere Inhaltsstoffe enthalten, wobei Tensid(en) und Pflegestoff(en) eine besondere Bedeutung zukommt.

Den Zubereitungen (A) und (B) wird bevorzugt zusätzlich eine oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, kationischen, zwitterionischen, amphoteren und nichtionischen Tensiden und Emulgatoren ausgewählt.

Den Zubereitungen (A) und (B) wird bevorzugt zusätzlich eine oberflächenaktive Substanz zugesetzt, wobei solche oberflächenaktive Substanzen je nach Anwendungsgebiet als Tenside oder als Emulgatoren bezeichnet werden: Sie sind bevorzugt aus zwitterionischen und nichtionischen Tensiden und Emulgatoren ausgewählt.

Erfindungsgemäß bevorzugte Mittel sind dadurch gekennzeichnet, daß das Mittel zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Weiterhin hat es sich als vorteilhaft erwiesen, wenn die Mittel weitere, nichtionogene grenzflächenaktive Stoffe, enthalten. Bevorzugte nichtionische Tenside sind beispielsweise Alkylenoxid-Anlagerungsprodukte an Fettalkohole und Fettsäuren mit jeweils 2 bis 30 Mol Ethylenoxid pro Mol Fettalkohol bzw. Fettsäure. Zubereitungen mit hervorragenden Eigenschaften werden ebenfalls erhalten, wenn sie als nichtionische Tenside Fettsäureester von ethoxyliertem Glycerin enthalten.

Alkylpolyglycoside sind besonders bevorzugt. Die Kombination Alkylpolyglycoside und zwitterionische Tenside ist ganz besonders bevorzugt.

In diesem Zusammenhang besonders bevorzugte nichtionische Tenside sind Alkylenoxid-Anlagerungsprodukte an Fettalkohole und Fettsäuren mit jeweils 2 bis 30 Mol Ethylenoxid pro Mol C₁₂-C₃₀-Fettalkohol bzw. C₁₂-C₃₀-Fettsäure. Alkylpolyglycoside sind ebenfalls besonders bevorzugte nichtionische Tenside.

Die nichtionischen und/oder zwitterionischen Tenside können in Anteilen von 0,1 bis 40 Gew.%, bevorzugt 1 bis 30 Gew.% und ganz besonders bevorzugt von 20 bis 25 Gew.%, jeweils bezogen auf die Gesamtmenge der Zubereitungen (A) und (b), eingesetzt werden.

Erfindungsgemäß besonders bevorzugte Mittel und Verfahren sind dadurch gekennzeichnet, daß die Anwendungsmischung - bezogen auf ihr Gewicht - einen Gesamttensidgehalt von 1,0 bis 50 Gew.-%, bevorzugt von 2,5 bis 45 Gew.-% und insbesondere von 5 bis 40 Gew.-% aufweist.

Als bevorzugte Fettbestandteile werden in diesem Zusammenhang die Bestandteile aus der Gruppe der C₁₂-C₃₀-Fettalkohole, der C₁₂-C₃₀-Fettsäuretriglyceride, der C₁₂-C₃₀-Fettsäuremonoglyceride, der C₁₂-C₃₀-Fettsäurediglyceride verstanden. Im Sinne der vorliegenden Erfindung werden explizit nur nichtionische Substanzen als Fettbestandteile betrachtet. Geladene Verbindungen wie beispielsweise Fettsäuren und ihre Salze werden nicht als Fettbestandteil verstanden.

Bei den C₁₂-C₃₀-Fettalkoholen kann es sich um gesättigte, ein- oder mehrfach ungesättigte, lineare oder verzweigte Fettalkohole mit 12 bis 30 C-Atomen handeln.

Beispiele für bevorzugte lineare, gesättigte C₁₂-C₃₀-Fettalkohole sind Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), Arachylalkohol (Eicosan-1-ol), Heneicosylalkohol (Heneicosan-1-ol) und/oder Behenylalkohol (Docosan-1-ol).

Bevorzugte lineare, ungesättigte Fettalkohole sind (9*Z*)-Octadec-9-en-1-ol (Oleylalkohol), (9*E*)-Octadec-9-en-1-ol (Elaidylalkohol), (9*Z*,12*Z*)-Octadeca-9,12-dien-1-ol (Linoleylalkohol), (9*Z*,12*Z*,15*Z*)-Octadeca-9,12,15-trien-1-ol (Linolenoylalkohol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Erucylalkohol ((13Z)-Docos-13-en-1-ol) und/oder Brassidylalkohol ((13*E*)-Docosen-1-ol).

Die bevorzugten Vertreter für verzweigte Fettalkohole sind 2-Octyl-dodecanol, 2-Hexyl-Dodecanol und/oder 2-Butyl-dodecanol.

Unter einem C₁₂-C₃₀-Fettsäuretriglycerid wird im Sinne der vorliegenden Erfindung der Triester des dreiwertigen Alkohols Glycerin mit drei Äquivalenten Fettsäure verstanden. Dabei können sowohl strukturgleiche als auch unterschiedliche Fettsäuren innerhalb eines Triglyceridmoleküls an den Esterbildungen beteiligt sein.

Es ist möglich, daß die Zubereitung (A) und/oder die Zubereitung (B) Fettstoff(e) enthält. Unter Fettsäuren sind erfindungsgemäß gesättigte oder ungesättigte, unverzweigte oder verzweigte, unsubstituierte oder substituierte C₁₂-C₃₀-Carbonsäuren zu verstehen. Ungesättigte Fettsäuren können einfach oder mehrfach ungesättigt sein. Bei einer ungesättigten Fettsäure kann bzw. können deren C-C-Doppelbindung(en) die Cis- oder Trans-Konfiguration aufweisen.

Es zeichnen sich die Fettsäuretriglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(*Z*)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure].

Die Fettsäuretriglyceride können auch natürlichen Ursprungs sein. Die in Sojaöl, Erdnußöl, Olivenöl, Sonnenblumenöl, Macadamianussöl, Moringaöl, Aprikosenkernöl, Marulaöl und/oder gegebenenfalls gehärtetem Rizinusöl vorkommenden Fettsäure-Triglyceride bzw. deren Gemische sind zum Einsatz im erfindungsgemäßen Produkt besonders geeignet.

Unter einem C₁₂-C₃₀-Fettsäuremonoglycerid wird der Monoester des dreiwertigen Alkohols Glycerin mit einem Äquivalent Fettsäure verstanden. Hierbei kann entweder die mittlere Hydroxygruppe des Glycerins oder die endständige Hydroxygruppe des Glycerins mit der Fettsäure verestert sein.

Es zeichnen sich die C₁₂-C₃₀-Fettsäuremonoglycerid durch besondere Eignung aus, bei welchen eine Hydroxygruppe des Glycerins mit einer Fettsäure verestert wird, wobei die Fettsäuren ausgewählt sind aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(*Z*)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] oder Nervonsäure [(15Z)-Tetracos-15-ensäure].

Unter einem C₁₂-C₃₀-Fettsäurediglycerid wird der Diester des dreiwertigen Alkohols Glycerin mit zwei Äquivalenten Fettsäure verstanden. Hierbei können entweder die mittlere und eine endständige Hydroxygruppe des Glycerins mit zwei Äquivalenten Fettsäure verestert sein, oder aber beide endständigen Hydroxygruppen des Glycerins sind mit jeweils einer Fettsäure verestert. Das Glycerin kann hierbei sowohl mit zwei strukturgleichen als auch mit zwei unterschiedlichen Fettsäuren verestert sein.

Es zeichnen sich die Fettsäurediglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(*Z*)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure].

Die anwendungsbereiten Färbemittel können weitere Hilfs- und Zusatzstoffe enthalten. So hat es sich als vorteilhaft erwiesen, wenn die Mittel mindestens ein zusätzliches Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind anionische, synthetische Polymere; kationische, synthetische Polymere; natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen; nichtionische, synthetische Polymere, wie Polyvinylalkohol oder Polyvinylpyrrolidinon; sowie anorganische Verdickungsmittel, insbesondere Schichtsilikate wie beispielsweise Bentonit, besonders Smektite, wie Montmorillonit oder Hectorit.

Auch zwitterionische Polymere können in den erfindungsgemäßen Mitteln enthalten sein. Bevorzugte zwitterionische Polymere werden ausgewählt aus der Gruppe der
- Copolymere aus Dimethyl-diallylammoniumsalzen und Acrylsäure, z.B. Polyquaternium-22,
- Copolymere aus Dimethyl-diallylammoniumsalzen und Methacrylsäure,
- Copolymere aus N,N,N-Trimethyl-3-[(2-methyl-1-oxo-2-propen-1-yl)amino]-1-propanaminiumsalzen und Acrylsäure,
- Copolymere aus N,N,N-Trimethyl-3-[(2-methyl-1-oxo-2-propen-1-yl)amino]-1-propanaminiumsalzen und Methacrylsäure,
- Copolymere aus N,N,N-Trimethyl-2-[(2-methyl-1-oxo-2-propen-1-yl)amino]-1-ethanaminiumsalzen und Acrylsäure,
- Copolymere aus N,N,N-Trimethyl-2-[(2-methyl-1-oxo-2-propen-1-yl)amino]-1-ethanaminiumsalzen und Methacrylsäure,
- Copolymere aus N,N,N-Trimethyl-3-[(2-methyl-1-oxo-2-propen-1-yl)amino]-1-propanaminiumsalzen, Acrylsäure und Acrylamid, z.B. Polyquaternium-53
- Copolymere aus N,N,N-Trimethyl-3-[(2-methyl-1-oxo-2-propen-1-yl)amino]-1-propanaminiumsalzen, Methacrylsäure und Acrylamid,
- Copolymere aus 1-Ethenyl-3-methyl-1H-imidazoliumsalzen, 1-Ethenyl-1H-imidazol, 1-Ethenyl-2-pyrrolidinon und Methacrylsäure, z.B. Polyquaternium-86,
- Copolymere aus 1-Ethenyl-3-methyl-1H-imidazoliumsalzen, 1-Ethenyl-1H-imidazol, 1-Ethenyl-2-pyrrolidinon und Acrylsäure.
Auch Gemische der vorgenannten bevorzugten zwitterionischen Polymere (c) können in den erfindungsgemäßen Mittel enthalten sein.

Die Zubereitungen (A) und (B) können weiterhin zusätzliche Wirk-, Hilfs- und Zusatzstoffe enthalten, um die Färbe- bzw. Aufhellleistung zu verbessern und weitere gewünschte Eigenschaften der Mittel einzustellen.

Es hat sich als vorteilhaft erweisen, wenn die Färbemittel (d.h. die Zubereitungen (A) und/oder (b)) insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure und Dipicolinsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Alle in den Zubereitungen (A) und (B) enthaltenen ionischen Komplexbildner fallen in die Gruppe der organischen Salze, ihre Einsatzkonzentrationen müssen daher bei der Berechnung der Gesamtmolalitäten M1 bzw. M2 berücksichtigt werden.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Wie bereits mehrfach erwähnt, werden die Zubereitungen (A) und (B) zu einer Anwendungsmischung vermischt, die auch als erfindungsgemäßes Mittel bezeichnet wurde. Obwohl die Zubereitungen (A) und (B) in jedem beliebigen Verhältnis miteinander gemischt werden können, hat es sich als bevorzugt herausgestellt, die Mengen an Zubereitung (A) und (B) in der Anwendungsmischung möglichst annähernd gewichtsgleich einzusetzen. Erfindungsgemäß besonders bevorzugte Mittel und Verfahren sind dadurch gekennzeichnet, daß die Zubereitung (A) und die Zubereitung (B) im Gewichtsverhältnis 2:1 bis 1:2, vzuw 15:10 bis 10:15 und insbesondere 1:1 zur Anwendungsmischung vermischt werden.

Es ist erfindungsgemäß bevorzugt, wenn die beschriebene Haarfärbezubereitung in einem geeigneten Spender aufgenommen und zur jeweiligen Verwendung abgegeben wird. Dabei erfolgt die Ausgabe der Haarfärbezubereitung grundsätzlich in Schaumform. Die schaumförmige Konsistenz der Zubereitung ist in diesem Zusammenhang sehr breit zu verstehen und umfasst jedwede Mischung von einer fließfähigen Zubereitung und einer gasförmigen Komponente. Insofern sind sowohl fließfähige als auch im wesentlichen feste, stabile Schaumkonsistenzen vom Erfindungsgegenstand umfasst.

Grundsätzlich umfasst ein erfindungsgemäßer Spender zumindest einen Vorratsbehälter zur Aufnahme wenigstens einer Komponente der Haarfärbezubereitung und eine Applikationsvorrichtung zur Ausgabe der Haarfärbezubereitung in Form von Schaum. Dabei ist der Vorratsbehälter insbesondere als tuben- oder flaschenförmiger Behälter gestaltet, während die Applikationsvorrichtung diesen einseitig offenen Behälter verschließt. Die eigentliche Zubereitungsabgabe wird bevorzugt mittels einer geeigneten Druckquelle bewirkt, welche in den Spender, insbesondere den Vorratsbehälter, integriert ist, oder aber mittels manuellem Druckaufbau durch den Benutzer der Haarfärbezubereitung selbst veranlasst.

Als Beispiel für erfindungsgemäße Spender mit integrierter Druckquelle sind Druckbehälter zu nennen, die üblicherweise im Behälterinneren entweder einen geeigneten Druckspeicher aufweisen, z. B. einen mechanischen, oder aber ein Treibmittel enthalten und auf diesem Wege das Innere des Behälters unter Druck setzen. Derartige Druckbehälter verfügen üblicherweise über geeignete Ventilvorrichtungen zur Ausgabe der im Inneren des Druckbehälters befindlichen Zubereitung während einer entsprechenden Ventilbetätigung. Solche Druckbehälter sind vor allem in Verbindung mit gasförmigen und/oder flüssigen Treibmitteln in Form von Aerosolspendern für unterschiedlichste kosmetische Anwendungen vorbekannt, z. B. Haarstylingspray, Haarfärbezubereitungen, Deospray, Rasierschaum/-gel etc..

Alternativ können erfindungsgemäß auch manuell betätigte Spender zum Einsatz kommen, die sich lediglich der Krafteinwirkung des Nutzers bedienen, um eine schaumförmige Zubereitungsabgabe zu bewirken. Bei diesen Bauformen kann vorteilhaft auf eine zusätzliche Druckquelle, z. B. Treibmittel, verzichtet werden, was vor allem aus Kosten- sowie Nachhaltigkeitsgründen wünschenswert ist. Solche durch manuelle Krafteinwirkung betätigbaren Schaumspender sorgen neben der Förderung der Haarfärbezubereitung aus dem Vorratsbehälter zur Abgabeöffnung hin parallel auch für eine entsprechende Aufschäumung der Haarfärbezubereitung. Während dieser Aufschäumung oder Schaumbildung wird grundsätzlich die Haarfärbezubereitung mit einer gasförmigen Komponente, insbesondere Luft, vermischt. Im Einzelnen ist dazu eine Schäumvorrichtung vorgesehen, die dies bewerkstelligt.

Gemäß einer ersten Variante eines manuell betätigbaren Spenders ist dieser als Schüttelspender ausgeführt, mit mindestens einem Vorratsbehälter zur Aufnahme der Haarfärbezubereitung und einer zugehörigen Abgabevorrichtung zur schaumförmigen Abgabe der Haarfärbezubereitung. Dabei ist die Abgabevorrichtung, insbesondere lösbar, mit dem Vorratsbehälter verbunden. Die eigentliche Schaumbildung erfolgt innerhalb des Schüttelspenders durch Agitation der Haarfärbezubereitung innerhalb des Vorratsbehälters. In sofern bildet der Schüttelspender in Verbindung der entsprechenden Spenderbewegung die oben genannte Schäumvorrichtung. Im Anschluss an diese Art der Aufschäumung kann dann die Abgabe der schaumförmigen Haarfärbezubereitung mittels der Abgabevorrichtung erfolgen.

Eine weitere sinnvolle Spendervariante ergibt sich durch Ausgestaltung als Quetsch- oder Squeeze-Schaumspender. Ein solcher Quetsch-Schaumspender besitzt neben dem mindestens einen Vorratsbehälter zur Aufnahme der Haarfärbezubereitung eine entsprechende Applikationsvorrichtung, innerhalb der die Aufschäumung sowie anschließende Abgabe der Haarfärbezubereitung erfolgt. Die eigentliche Förderung der Haarfärbezubereitung aus dem Vorratsbehälter wird mittels Krafteinwirkung auf die flexible Vorratsbehälterwandung bewirkt. Dabei sorgt die reversible Verformung der Vorratsbehälterwandung für einen Druckaufbau im Inneren des Vorratsbehälters, so daß als Folge die Haarfärbezubereitung aus dem Vorratsbehälter getrieben wird. Dazu ist es notwendig die Vorratsbehälterwandung hinreichend flexibel bzw. reversibel verformbar zu gestalten. Dies wird gewährleistet durch eine anwendungsbezogen zielgerichte Auslegung der Dicke der Vorratsbehälterwandung in Verbindung mit einer geeigneten Materialauswahl für die Vorratsbehälterwandung. Bevorzugt wird die Vorratsbehälterwandung eines entsprechenden Quetsch-Schaumspenders aus einem Polyolefin, wie beispielsweise Polypropylen (PP), high density Polyethylen (HDPE), medium density Polyethylen (MDPE), low density Polyethylen (LDPE), linear low density Polyethylen (LLDPE), ausgeführt. Darunter ist Polypropylen (PP) bevorzugt geeignet.

Die Applikationsvorrichtung eines solchen Quetsch-Schaumspenders umfasst zur Aufschäumung der Haarfärbezubereitung auch eine entsprechende Schäumvorrichtung. Die Schäumvorrichtung ist in der Lage eine Zubereitungsmenge mit einer Gasmenge im geeigneten Dosierverhältnis zu vermischen, um die gewünschte Schaumkonsistenz der Haarfärbezubereitung auszubilden. Üblicherweise wird hierzu ein eingezogener Zubereitungsstrom mit einem eingezogenen Gasstrom innerhalb einer Mischkammer der Schäumvorrichtung zusammen geführt und dort durch strömungstechnische Verwirbelung vermengt. Besonders bevorzugt wird als gasförmige Komponente zur Schaumbildung Luft verwendet, die entweder unmittelbar aus der Vorratsbehälter oder der Umgebung eingezogen wird.

Analog kann der Spender auch als Pump-Schaumspender ausgebildet sein mit mindestens einer Vorratsbehälter zur Aufnahme der Haarfärbezubereitung sowie einer Applikationsvorrichtung, wobei in diesem Fall die Applikationsvorrichtung eine Pumpvorrichtung zur Förderung sowohl der Haarfärbezubereitung als der gasförmigen Komponente, bevorzugt Luft, aufweist und darüber hinaus eine entsprechende Schäumvorrichtung umfasst.

Bei Verwendung der genannten Spendervarianten in Verbindung mit mehrkomponentigen Haarfärbezubereitungen, mit untereinander unverträglichen Einzelkomponenten (z. B. mehrkomponentige oxidative Haarfärbezubereitungen), muss sichergestellt werden, daß die Einzelkomponenten bis zur eigentlichen Zubereitungsanwendung getrennt bevorratet werden. Dies geschieht vorteilhaft durch Verwendung mehrerer Vorratsbehälter zur Aufnahme der jeweiligen Einzelkomponenten der Haarfärbezubereitung, wobei jeder Vorratsbehälter zur Abgabe der Haarfärbezubereitung jeweils in Fluidverbindung mit der zugehörigen Applikationsvorrichtung steht. Damit erfolgt eine Vermischung der Einzelkomponenten der Haarfärbezubereitung erst unmittelbar bei der Ausgabe der Zubereitung aus dem erfindungsgemäßen Spender bzw. bei der eigentlichen Zubereitungsverwendung. Beispielsweise kann ein erfindungsgemäßer Pump-Schaumspender auch mit zwei oder mehreren Vorratsbehältern für mehrere Zubereitungskomponenten ausgestattet sein.

Anderseits kann eine mehrkomponentige Haarfärbezubereitung erfindungsgemäß auch mit einer der oben beschriebenen Spendervarianten mit nur einem Vorratsbehälter und einer Applikationsvorrichtung angewendet werden. Dafür ist der Vorratsbehälter derart gestaltet, daß er sich wieder verschließbar öffnen lässt. Idealerweise ist der Vorratsbehälter mittels der Applikationsvorrichtung verschlossen, wobei die Applikationsvorrichtung lösbar mit dem Vorratsbehälter verbunden ist, beispielsweise über eine Schraub- oder Rastverbindung. Dies eröffnet die Möglichkeit den Vorratsbehälter bereits bei der Herstellung mit einer Komponente der Haarfärbezubereitung vorzukonfektionieren und weitere Komponenten der Haarfärbezubereitung erst kurz vor der eigentlichen Anwendung der Haarfärbezubereitung in den Vorratsbehälter zuzugeben. Hierbei sind die weiteren Komponenten der Haarfärbezubereitung dem gesamten Haarfärbeprodukt in Form eines Kit innerhalb von geeigneten separaten Behältern beigegeben und werden vom Nutzer unmittelbar vor der Anwendung der Haarfärbezubereitung im Vorratsbehälter gemischt.

Darüber hinaus können zusätzlich ein oder mehrere poröse Einsatzelemente eingesetzt werden, um die innerhalb der Schäumvorrichtung erzielbare Schaumkonsistenz positiv zu beeinflussen. Solche porösen Einsatzelemente sind beispielsweise schwamm- oder netzartig gestaltet und sind innerhalb der Schäumvorrichtung an geeigneter Stelle im Strömungskanal für die Haarfärbezubereitung positioniert, etwa unmittelbar stromaufwärts der Spenderabgabeöffnung. Das poröse Einsatzelement gestattet somit die Durchströmung der Haarfärbezubereitung und sorgt infolge strömungstechnischer Verwirbelung für eine feinere und homogenere Schaumkonsistenz. In Abhängigkeit von der jeweiligen Gestaltung des porösen Einsatzelementes lässt sich somit die Schaumkonsistenz direkt beeinflussen. Bei Verwendung eines netzartigen Einsatzelementes erweist es sich als sinnvoll das netzartige Einsatzelement bevorzugt mit einer Lochdichte von 50 bis 220 mesh (mesh = Anzahl der Löcher pro Inch), besonders bevorzugt von 90 bis 200 mesh, ganz besonders bevorzugt von 125 bis 175 mesh auszubilden. Bei Verwendung mehrerer netzartiger Einsatzelemente können auch Einsatzelemente mit unterschiedlichen Lochdichten zum Einsatz gelangen. Hierbei weist das erste, stromaufwärts positionierte netzartige Einsatzelement bevorzugt eine Lochdichte von 50 bis 220 mesh (mesh = Anzahl der Löcher pro Inch), besonders bevorzugt von 90 bis 200 mesh, ganz besonders bevorzugt von 125 bis 175 mesh auf. Das zweite, stromabwärts positionierte Netz weist bevorzugt eine Lochdichte von 160 bis 280 mesh, besonders bevorzugt von 175 bis 245 mesh und ganz besonders bevorzugt von 180 bis 220 mesh, auf. Letztlich wird die Anzahl der verwendeten porösen Einsatzelemente wie auch deren spezifische Lochdichte bzw. deren Porösitätseigenschaften in Abhängigkeit vom jeweiligen Anwendungsfall zielgerichtet ausgelegt.

Die Anwendungstemperaturen des resultierenden Schaums können in einem Bereich zwischen 15 und 40 °C liegen. Nach einer Einwirkungszeit von 5 bis 45 Minuten wird das Haarfärbemittel durch Ausspülen von dem zu färbenden Haar entfernt.

Die Anwendungsmischung wird als Schaum aus einem geeigneten Gefäß auf das Haar aufgebracht und dort gattungsgemäß zur Haarfärbung verwendet. Bvesonders bevorzugte erfindungsgemäße Verfahren sind dadurch gekennzeichnet, daß der Spender ein Einkammer-Quetschschaumspender oder ein Einkammer-Pumpschaumspender ist, aus dem die Anwendungsmischung als Schaum ausgebracht wird, anschließend der so erhaltene Schaum auf den Fasern verteilt wird, dann für eine Zeit von 1 bis 60 Minuten, vorzugsweise von 5 bis 40 Minuten, auf den Fasern verbleibt und anschließend aus den Fasern ausgewaschen wird.

## Patentansprüche

1. Mittel zum Färben keratinischer Fasern, enthaltend mindestens zwei getrennt voneinander verpackte Zubereitungen (A) und (B) sowie gegebenenfalls eine weitere getrennt von (A) und (B) verpackte Zubereitung (C), die unmittelbar vor der Anwendung zu einer Anwendungsmischung vermischt werden, wobei
i. Zubereitung (A)
a) mindestens ein Oxidationsfarbstoffvorprodukt und
b) mindestens ein Kaliumsalz einer Fettsäure enthält und
ii. Zubereitung (B) fließfähig ist und - bezogen auf ihr Gewicht -
c) mindestens ein Oxidationsmittel und
d) Xanthan
enthält.

2. Verfahren zum Färben keratinischer Fasern, bei dem mindestens zwei getrennt voneinander verpackte Zubereitungen (A) und (B) sowie gegebenenfalls eine weitere getrennt von (A) und (B) verpackte Zubereitung (C), unmittelbar vor der Anwendung zu einer Anwendungsmischung vermischt und aus einem Spender in Form eines Schaumes ausgebracht werden, **dadurch gekennzeichnet, daß**
i. Zubereitung (A)
a) mindestens ein Oxidationsfarbstoffvorprodukt und
b) mindestens ein Kaliumsalz einer Fettsäure enthält und
ii. Zubereitung (B) fließfähig ist und - bezogen auf ihr Gewicht -
a) mindestens ein Oxidationsmittel und
b) Xanthan
enthält.

3. Mittel nach Anspruch 1 oder Verfahren nach Anspruch 2, **dadurch gekennzeichnet, daß** die Zubereitung (A) - bezogen auf ihr Gewicht - 0,1 bis 10 Gew.-%, vorzugsweise 0,15 bis 7,5 Gew.-%, weiter bevorzugt 0,2 bis 6 Gew.-%, besonders bevorzugt 0,25 bis 5,5 Gew.-% und insbesondere 0,3 bis 5 Gew.-% mindestens eines Kaliumsalzes mindestens einer Fettsäure enthält.

4. Mittel nach einem der Ansprüche 1 oder 3 oder Verfahren nach einem der Ansprüche 2 oder 3, **dadurch gekennzeichnet, daß** die Zubereitung (A) einen pH-Wert von 8 bis 12, vorzugsweise von 8,5 bis 11,5 und insbesondere von 9 bis 11 aufweist.

5. Mittel nach einem der Ansprüche 1 oder 3 bis 4 oder Verfahren nach einem der Ansprüche 2 bis 4, **dadurch gekennzeichnet, daß** die Zubereitung (A) - bezogen auf ihr Gewicht - 0,1 bis 15 Gew.-%, vorzugsweise 0,5 bis 10 Gew.-%, weiter bevorzugt 2 bis 9 Gew.-%, besonders bevorzugt 3 bis 8 Gew.-% und insbesondere 4 bis 7 Gew.-% mindestens eines Alkanolamins aus primären, sekundären oder tertiären Aminen mit einem C₂-C₆-Alkylgrundkörper, der mindestens eine Hydroxylgruppe trägt, bevorzugt ausgewählt aus der Gruppe, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethyl-ethanolamin, Triethanolamin, Diethanolamin und Triisopropanolamin und besonders bevorzugt ausgewählt aus Monoethanolamin, enthält.

6. Mittel nach einem der Ansprüche 1 oder 3 bis 5 oder Verfahren nach einem der Ansprüche 2 bis 5, **dadurch gekennzeichnet, daß** die Zubereitung (B) - bezogen auf ihr Gewicht - 0,01 bis 4 Gew.-%, vorzugsweise 0,02 bis 3,5 Gew.-%, weiter bevorzugt 0,03 bis 3 Gew.-%, besonders bevorzugt 0,04 bis 2,5 Gew.-% und insbesondere 0,05 bis 2 Gew.-% Xanthan enthält.

7. Mittel nach einem der Ansprüche 1 oder 3 bis 6 oder Verfahren nach einem der Ansprüche 2 bis 6, **dadurch gekennzeichnet, daß** die Zubereitung (B) einen pH-Wert von 1 bis 6, vorzugsweise von 1,5 bis 5,5 und insbesondere von 2 bis 5 aufweist.

8. Mittel nach einem der Ansprüche 1 oder 3 bis 7 oder Verfahren nach einem der Ansprüche 2 bis 7, **dadurch gekennzeichnet, daß** die Zubereitung (B) - bezogen auf ihr Gewicht - 0,1 bis 15 Gew.-%, vorzugsweise 0,25 bis 12 Gew.-%, weiter bevorzugt 0,5 bis 10 Gew.-% und insbesondere 3 bis 8 Gew.-% Wasserstoffperoxid (berechnet als 100%iges H₂O₂) enthält.

9. Mittel nach einem der Ansprüche 1 oder 3 bis 8 oder Verfahren nach einem der Ansprüche 2 bis 8, **dadurch gekennzeichnet, daß** die Zubereitung (A) und die Zubereitung (B) im Gewichtsverhältnis 2:1 bis 1:2, vzuw 15:10 bis 10:15 und insbesondere 1:1 zur Anwendungsmischung vermischt werden.

10. Mittel nach einem der Ansprüche 1 oder 3 bis 9 oder Verfahren nach einem der Ansprüche 2 bis 9, **dadurch gekennzeichnet, daß** die Anwendungsmischung - bezogen auf ihr Gewicht - einen Gesamttensidgehalt von 1,0 bis 50 Gew.-%, bevorzugt von 2,5 bis 45 Gew.-% und insbesondere von 5 bis 40 Gew.-% aufweist.

11. Verfahren nach einem der Ansprüche 2 bis 10, **dadurch gekennzeichnet, daß** der Spender ein Einkammer-Quetschschaumspender oder ein Einkammer-Pumpschaumspender ist, aus dem die Anwendungsmischung als Schaum ausgebracht wird, anschließend der so erhaltene Schaum auf den Fasern verteilt wird. dann für eine Zeit von 1 bis 60 Minuten, vorzugsweise von 5 bis 40 Minuten, auf den Fasern verbleibt und anschließend aus den Fasern ausgewaschen wird.

## Claims

1. An agent for coloring keratin fibers, containing at least two preparations (A) and (B) that are packaged separately from one another, and optionally a further preparation (C) that is packaged separately from (A) and (B), which preparations are mixed immediately before application to form an application mixture, wherein
i. preparation (A) contains
a) at least one oxidation dye precursor and
b) at least one potassium salt of a fatty acid
and
ii. preparation (B) is flowable and, based on the weight thereof, contains
c) at least one oxidizing agent and
d) xanthan
gum.

2. A method for coloring keratin fibers, in which at least two preparations (A) and (B) that are packaged separately from one another, and optionally a further preparation (C) that is packaged separately from (A) and (B), are mixed immediately before application to form an application mixture and are applied from a dispenser in the form of a foam, **characterized in that**
i. preparation (A) contains
a) at least one oxidation dye precursor and
b) at least one potassium salt of a fatty acid
and
ii. preparation (B) is flowable and, based on the weight thereof, contains
a) at least one oxidizing agent and
b) xanthan
gum.

3. The agent according to claim 1 or the method according to claim 2, **characterized in that** preparation (A) contains, based on the weight thereof, from 0.1 to 10 wt.%, preferably from 0.15 to 7.5 wt.%, more preferably from 0.2 to 6 wt.%, particularly preferably from 0.25 to 5.5 wt.%, and in particular from 0.3 to 5 wt.%, of at least one potassium salt of at least one fatty acid.

4. The agent according to one of claims 1 or 3 or the method according to one of claims 2 or 3, **characterized in that** preparation (A) has a pH of from 8 to 12, preferably of from 8.5 to 11.5, and in particular of from 9 to 11.

5. The agent according to one of claims 1 or 3 to 4 or the method according to one of claims 2 to 4, **characterized in that** preparation (A) contains, based on the weight thereof, from 0.1 to 15 wt.%, preferably from 0.5 to 10 wt.%, more preferably from 2 to 9 wt.%, particularly preferably from 3 to 8 wt.%, and in particular from 4 to 7 wt.% of at least one alkanolamine from primary, secondary or tertiary amines having a C₂-C₆ alkyl parent substance that bears at least one hydroxyl group, preferably selected from the group formed of 2-aminoethan-1-ol (monoethanolamine), 3-aminopropan-1-ol, 4-aminobutan-1-ol, 5-aminopentan-1-ol, 1-aminopropan-2-ol (monoisopropanolamine), 1-aminobutan-2-ol, 1-aminopentan-2-ol, 1-aminopentan-3-ol, 1aminopentan-4-ol, 2-amino-2-methylpropanol, 2-amino-2-methylbutanol, 3-amino-2-methylpropan-1-ol, 1-amino-2-methylpropan-2-ol, 3-aminopropane-1,2-diol, 2-amino-2-methylpropane-1,3-diol, 2-amino-2-ethyl-1,3-propanediol, N,N-dimethyl-ethanolamine, triethanolamine, diethanolamine and triisopropanolamine, and particularly preferably selected from monoethanolamine.

6. The agent according to one of claims 1 or 3 to 5 or the method according to one of claims 2 to 5, **characterized in that** preparation (B) contains, based on the weight thereof, from 0.01 to 4 wt.%, preferably from 0.02 to 3.5 wt.%, more preferably from 0.03 to 3 wt.%, particularly preferably from 0.04 to 2.5 wt.%, and in particular from 0.05 to 2 wt.%, of xanthan gum.

7. The agent according to one of claims 1 or 3 to 6 or the method according to one of claims 2 to 6, **characterized in that** preparation (B) has a pH of from 1 to 6, preferably of from 1.5 to 5.5, and in particular of from 2 to 5.

8. The agent according to one of claims 1 or 3 to 7 or the method according to one of claims 2 to 7, **characterized in that** preparation (B) contains, based on the weight thereof, from 0.1 to 15 wt.%, preferably from 0.25 to 12 wt.%, more preferably from 0.5 to 10 wt.%, and in particular from 3 to 8 wt.%, of hydrogen peroxide (calculated as 100% H₂O₂).

9. The agent according to one of claims 1 or 3 to 8 or the method according to one of claims 2 to 8, **characterized in that** preparation (A) and preparation (B) are mixed together in a weight ratio of from 2:1 to 1:2, preferably from 15:10 to 10:15, and in particular 1:1, to form the application mixture.

10. The agent according to one of claims 1 or 3 to 9 or the method according to one of claims 2 to 9, **characterized in that** the application mixture has, based on the weight thereof, a total surfactant content of from 1.0 to 50 wt.%, preferably from 2.5 to 45 wt.%, and in particular from 5 to 40 wt.%.

11. The method according to one of claims 2 to 10, **characterized in that** the dispenser is a single-chamber squeeze foam dispenser or a single-chamber pump foam dispenser, from which the application mixture is applied as a foam, then the foam obtained in this way is distributed onto the fibers, remains on the fibers for a period of from 1 to 60 minutes, preferably from 5 to 40 minutes, and is then washed out of the fibers.

## Revendications

1. Agent destiné à colorer des fibres de kératine, contenant au moins deux préparations (A) et (B) conditionnées séparément et, éventuellement une autre préparation (C) conditionnée séparément de (A) et (B), qui sont mélangées immédiatement avant utilisation pour donner un mélange prêt à l'emploi, où
i. la préparation (A) contient
a) au moins un précurseur de colorant d'oxydation et
b) au moins un sel de potassium d'un acide gras
et où
ii. la préparation (B) est fluide et contient - rapporté à son poids -
c) au moins un oxydant et
d) du xanthane.

2. Procédé de coloration de fibres de kératine, dans lequel au moins deux préparations (A) et (B) conditionnées séparément et éventuellement une autre préparation (C) conditionnée séparément de (A) et (B), sont mélangées immédiatement avant utilisation pour obtenir un mélange prêt à l'emploi, et sont délivrées par un distributeur sous forme d'une mousse, **caractérisé en ce que**
i. la préparation (A) contient
a) au moins un précurseur de colorant d'oxydation et
b) au moins un sel de potassium d'un acide gras
et
ii. la préparation (B) est fluide et contient - rapporté à son poids -
a) au moins un oxydant et
b) du xanthane.

3. Agent selon la revendication 1 ou procédé selon la revendication 2, **caractérisé en ce que** la préparation (A) contient - rapporté à son poids - de 0,1 à 10 % en poids, de préférence de 0,15 à 7,5 % en poids, plus préférentiellement de 0,2 à 6 % en poids, de manière particulièrement préférée de 0,25 à 5,5 % en poids et en particulier de 0,3 à 5 % en poids d'au moins un sel de potassium d'au moins d'un acide gras.

4. Produit selon une des revendications 1 ou 3 ou procédé selon une des revendications 2 ou 3, **caractérisé en ce que** la préparation (A) présente un pH de 8 à 12, de préférence de 8,5 à 11,5 et en particulier de 9 à 11.

5. Agent selon une des revendications 1 ou 3 à 4 ou procédé selon une des revendications 2 à 4, **caractérisé en ce que** la préparation (A) contient - rapporté à son poids - de 0,1 à 15 % en poids, de préférence de 0,5 à 10 % en poids, plus préférentiellement de 2 à 9 % en poids, de manière particulièrement préférée de 3 à 8 % en poids et en particulier de 4 à 7 % en poids d'au moins une alcanolamine constituée d'amines primaires, secondaires ou tertiaires avec un squelette de C₂-₆-alkyle qui contient au moins un groupe hydroxyle, choisie dans le groupe constitué de 2-aminoéthan-1-ol (monoéthanolamine), 3-aminopropan-1-ol, 4-aminobutan-1-ol, 5-aminopentan-1-ol, 1-aminopropan-2-ol (monoisopropanolamine), 1-aminobutan-2-ol, 1-aminopentan-2-ol, 1-aminopentan-3-ol, 1-aminopentan-4-ol, 2-amino-2-méthyl-propanol, 2-amino-2-méthylbutanol, 3-amino-2-méthylpropan-1-ol, 1-amino-2-méthylpropan-2-ol, 3-aminopropan-1,2-diol, 2-amino-2-méthylpropan-1,3-diol, 2-amino-2-éthyl-1,3-propandiol, N,N-diméthyl-éthanolamine, triéthanolamine, diéthanolamine et triisopropanolamine et de manière particulièrement préférée la monoéthanolamine.

6. Agent selon une des revendications 1 ou 3 à 5 ou procédé selon une des revendications 2 à 5, **caractérisé en ce que** la préparation (B) contient - rapporté à son poids - de 0,01 à 4 % en poids, de préférence de 0,02 à 3,5 % en poids, plus préférentiellement de 0,03 à 3 % en poids, de manière particulièrement préférée de 0,04 à 2,5 % en poids et en particulier de 0,05 à 2 % en poids de xanthane.

7. Agent selon une des revendications 1 ou 3 à 6 ou procédé selon une des revendications 2 à 6, **caractérisé en ce que** la préparation (B) présente un pH de 1 à 6, de préférence de 1,5 à 5,5 et en particulier de 2 à 5.

8. Agent selon une des revendications 1 ou 3 à 7 ou procédé selon une des revendications 2 à 7, **caractérisé en ce que** la préparation (B) contient, - rapporté à son poids - de 0,1 à 15 % en poids, de préférence 0,25 à 12 % en poids, plus préférentiellement de 0,5 à 10 % en poids et en particulier de 3 à 8 % en poids de peroxyde d'hydrogène (calculé comme 100 % de H₂O₂).

9. Agent selon une des revendications 1 ou 3 à 8 ou procédé selon une des revendications 2 à 8, **caractérisé en ce que** la préparation (A) et la préparation (B) sont mélangées dans un rapport en poids de 2:1 à 1:2, préférentiellement de 15:10 à 10:15 et en particulier de 1:1 pour donner un mélange prêt à l'emploi.

10. Agent selon une des revendications 1 ou 3 à 9 ou procédé selon une des revendications 2 à 9, **caractérisé en ce que** le mélange prêt à l'emploi présente - rapporté à son poids - une teneur totale en tensioactifs de 1,0 à 50 % en poids, préférentiellement de 2,5 à 45 % en poids et en particulier de 5 à 40 % en poids.

11. Procédé selon une des revendications 2 à 10, **caractérisé en ce que** le distributeur est un distributeur de mousse par pression à chambre unique ou un distributeur de mousse par pompage à chambre unique, d'où le mélange prêt à l'emploi est extrait sous forme de mousse, puis la mousse ainsi obtenue est répartie sur les fibres, puis reste sur les fibres pendant une durée de 1 à 60 minutes, de préférence de 5 à 40 minutes, puis est éliminée des fibres par rinçage.
